(19) **Europäisches Patentamt / European Patent Office / Office européen des brevets**

(11) **EP 4 135 832 B1**

(12) **EUROPEAN PATENT SPECIFICATION**

(45) Date of publication and mention
of the grant of the patent:
**02.07.2025 Bulletin 2025/27**

(21) Application number: **21722950.9**

(22) Date of filing: **07.05.2021**

(51) International Patent Classification (IPC):
*A61N 1/04* *(2006.01)* *A61N 1/32* *(2006.01)*
*A61N 1/20* *(2006.01)*

(52) Cooperative Patent Classification (CPC):
**A61N 1/0468; A61N 1/326;** A61N 1/205

(86) International application number:
**PCT/EP2021/062204**

(87) International publication number:
**WO 2021/224480 (11.11.2021 Gazette 2021/45)**

(54) **DEVICE FOR ELECTRICAL WOUND CARE**

VORRICHTUNG ZUR ELEKTRISCHEN WUNDVERSORGUNG

DISPOSITIF POUR LE TRAITEMENT ÉLECTRIQUE DES PLAIES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.05.2020 EP 20173482**

(43) Date of publication of application:
**22.02.2023 Bulletin 2023/08**

(73) Proprietor: **Vanquish Innovation ApS
2920 Charlottenlund (DK)**

(72) Inventors:
 • **SIESBYE, Valdemar
  1573 København V (DK)**
 • **SØRENSEN, Henrik Riehm
  8600 Silkeborg (DK)**
 • **MCCAIG, Colin Darnley
  Aberdeen, Aberdeenshire AB 51 8LX (GB)**

(74) Representative: **AWA Denmark A/S
Strandgade 56
1401 Copenhagen K (DK)**

(56) References cited:
 **WO-A1-2015/157725**   **US-A- 5 018 524**
 **US-A1- 2004 267 333**   **US-A1- 2009 132 010**
 **US-B1- 6 535 767**

**Description**

**Field of the invention**

The present invention relates to electro-therapy of wounds. Specifically, a device for electro-therapy of wounds is provided. The device of the invention is useful for treating any kind of wound and is especially useful for facilitating the healing of difficult to heal or chronic wounds.

**Background of the invention**

Chronic wounds, such as diabetic, venous, or pressure ulcers place an immense financial cost on the healthcare system, while also causing pain and physical stress on the afflicted patients. The dominant way to heal chronic wounds today is to apply a bandage or wound dressing, i.e. a bandage with healing factors applied in it, in combination with antibiotic treatments to avoid or combat infection. Such treatments are lengthy and are in many cases not particularly efficient and it may therefore take months or even years to heal chronic wounds, during which time the quality of life for the patient (and their family) will be reduced. The cost for the healthcare system for these treatments are also high as many patients suffering from chronic wounds are elderly patients with compromised immune systems, for whom the wound provides a highly increased risk of infection, thus leading to further complication. Other types of wounds, such as surgical wounds, may also suffer from the same complications as mentioned above and in such cases the importance of reducing infection in the wounds are equally important.

Electro-therapy has been documented to promote blood flow and accelerate wound healing, thus making it an attractive alternative or supplementary treatment. Because the treatment duration for healing wounds and promoting blood circulation require longer treatment periods for the body to respond, a preferred way of carrying out the treatment is by implementing electrodes into dressings, such that the dressing may be used to cover the wound while the patient continues their daily routines during the electro-therapy treatment. The following electro-therapies constitute relevant examples from the art.

WO2005032652A1 relates to a dressing for treating damaged tissue, incorporating a pair of electrodes and a conductive gel between the electrodes. An electric current passes between the electrodes through the gel to repair the damaged tissue. Sensors can be incorporated into the dressing along with a control unit. The control unit can vary current supplied to the electrodes according to environmental parameters detected by the sensors. Alternatively, one or more pre-defined programs can be stored in the control unit for supplying an alternating current to the electrodes with a varying amplitude, frequency and waveform.

WO2009060211A1 relates to an apparatus providing electrical stimulation to tissue from a control unit and at least one electrode connected to the control unit. A fixation element holds the at least one electrode against the tissue and also holds the control unit in a fixed position with respect to the tissue. The control unit is configured to supply electrical current to the at least one electrode for stimulation of the tissue.

WO02098502 relates to an electrode system for facilitating the healing of a wound. The device has a supporting structure, and two electrodes attached to the supporting structure. One of the electrodes surrounds the other on the supporting structure. The electrodes are applied to the wound and a voltage potential is applied across the electrodes, so that a current is caused to flow between them and through the wound.

WO2008013936 relates to an electrode system for applying therapy to a wound. The system comprises two electrodes: the first electrode is configured at least in part to be applied to the wound and the second electrode is configured at least in part to be applied to one of skin surrounding the wound and an outer portion of the wound. The system furthermore comprises one or more feedback sensors to detect the effectiveness of the treatment and adjust the electrical stimulation. The feedback sensors are connected to a control module, which can adjust the voltage based on the sensors output. The feedback sensor may be a voltage sensor.

US2004267333A1 relates to a device and a method for generating an electrical signal for use in biomedical applications. The device includes means for creating a complex electrical signal and conductive means for coupling the signal to a human or animal body, cell or tissue culture, in order to relieve pain, stimulate healing or growth.

US6535767B1 relates to an apparatus for generating an electrical signal for use in biomedical applications. The apparatus is configured to output a repeating succession of a burst of rectangular waves, an equalizing pulse (if needed) to cancel net DC, and a rest period of no signal.

US2009132010A1 relates to a system and method for generating an electrical signal for use in biomedical applications which has power efficient features, support battery powered operation and, support a reduced risk of shock hazard. The system may include a controller for generating one or more control signals operable to control pulse generating and waveform processing circuits.

US5018524A relates to an apparatus and a method for generating vital information signals which are formed by a pulse train made up of bursts of the same width and distance, wherein each burst is made up of a given number of pulses of a specific frequency.

In light of the devices disclosed in the prior art there is still a need for an improved electro-therapy, which facilitates faster and efficient healing of wounds.

**Disclosure of the invention**

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes. The device for electro-therapy, comprises a controlled voltage source connected to at least two stimuli electrodes (204a, 204b), the stimuli electrodes being configured to be electrically in contact with skin having a wound, which the stimuli electrodes are configured to be electrically in contact with skin from the edge of the wound to a distance at maximum 50 mm from the edge of the wound, and in that the controlled voltage source is configured to output a pulsed direct current through the wound with a frequency of at least 50 kHz, and in that the pulsed direct current has a duty cycle in the range of 1-10%

Electrical contact can be any kind of contact made between an electrode and the tissue in question, which enables the conduction of a current in the skin. For example, it may be a direct contact between a conductor of the electrode and the tissue. It could also be indirect, such as when a conductive medium is laid between the electrode and the tissue. Without limiting the invention to a specific medium, an example of such medium could be conductive hydrogels, which are well known from their use in electrocardiography and in ultrasound applications. For a given wound, a conductive hydrogel may cover the skin surrounding the wound.

The device is useful in a method for treating any kind of wound and may especially be useful for, but is not limited to, facilitating the healing of chronic wounds. Such chronic wounds may arise from a surgical infection, a diabetic infection, a surgical wound such as from a c-section, a trauma wound, a venous infection, a pressure ulcer, a venous ulcer, a diabetic foot ulcer, a diabetic foot, or an arterial ulcer. The device may also be used in a precautionary treatment to reduce the risk of infection upfront.

The electrodes may be of any material with good conductive properties, such as a metal such as platinum, gold, cobber, silver, iron or led, or an alloy, or other conductive materials such as carbon.

The device of the present disclosure allows for a current to run through a wound, when the stimuli electrodes are put into electrical contact with the skin surrounding the wound. The current is supplied through the stimuli electrodes, and the current may run from one of the stimuli electrodes, through the skin, through the wound, through the skin again, and to another stimuli electrode. When a current is applied to the wound using the device, the electrical field formed in the wound may improve compromised physiological processes and thereby function to accelerate wound healing, and as such the device provides electrotherapy for a wound.

Being able to apply an electrotherapy to a wound through the skin surrounding the wound may be preferred over applying electrotherapy directly on the wound itself, as placing the electrodes inside or onto the wound may interfere with wound crust formation and how wound treatments are carried out today. For example, in conventional wound care, dressings may be applied to the wound to drain the wound of moisture or many other functions. To be effective, such dressing often need to be arranged so that they are in contact with the wound itself. Arranging electrodes on, inside or onto the wound therefore hinders the access of the dressings contact to the wound and thereby the dressings effects. As the device of the present disclosure may be used for applying electrotherapy to a wound through the skin surrounding the wound, the device does not hinder the use of dressings that are arranged to be in contact with the wound itself. Hence the device of the present disclosure enables that both electrotherapy and conventional wound care may be carried out simultaneously. Arranging the stimuli electrodes on or onto skin surrounding the wound may furthermore enable easy installation of the stimuli electrodes and may afford less pain for the patient when arranged and removed when compared to arranging and removing electrodes, which has been arranged on, onto or inside the wound itself. The ease of arranging the stimuli electrodes is of further relevance to the caregiver responsible for preparing the dressing since little or no training is required for applying and configuring the electrodes on or onto the skin surrounding the wound.

In the context of the present application a pulsed direct current should be understood as a direct current, which alternates between periods of no voltage to a period with voltage, such as alternating between 0V and 5V, between 0V and 10V, between 0V and 20V, and between 0V and 50V.

The frequency of the pulsed direct current refers to how many cycles of periods with and without voltage per second the pulsed direct current has. For instance, if the pulsed direct current has a frequency of 2 Hz, the pulsed direct current will have two periods with voltage and two periods without voltage, such as 0.25 seconds of 2V, then 0.25 seconds of 0V, then 0.25 seconds of 2V and then 0.25 seconds of 0V. The periods with or without voltage do not need to be of the same duration, and the relationship between periods with and without voltage may be in the range of 1:1.1 to 1:20, 1:1.5 to 1:10, or 1:2 to 1:5. Furthermore, the rise and fall in voltage may take any suitable form, such as a sawtooth wave, a square wave, a triangular wave, a sine wave, or a combination thereof. In an embodiment, the voltage takes the form of a square wave.

The DC signal may for instance be in a square-wave form (pulse width modulation, where the frequency may for instance be 50 kHz, the amplitude 10V and the duty cycle 10%). When applied to a wound, the electrical pulses may be band-limited due to the impedances, such that the electrical signal going through the wound becomes mores sinusoidal in shape. The signal may also be intentionally band-limited, or by other means shaped such that the rise time is extended in comparison with for instance a perfect square-wave signal. The longer rise time alleviates uncomfortable sensations, which arise from sudden electrical stimulation. The current is preferably mono pulsed DC and will for a given time, for instance 1 min to 1 hour, run in a first direction through the electrodes and the wound. After this period, the current may be applied in the opposite direction of the first current direction. The purpose of the mono pulsed DC signal is to mimic the natural electrical stimulation occurring in wounds, which may not be present in e.g. chronic wounds or other severe wound types, such as those seen for older patients or patients with chronic disease. In the context of the present inventive concept, the terms "mono" or "monophasic" as used in the context of pulsed direct current (DC) are used interchangeably and means that the current travels in one direction as opposed to a biphasic system in which the current is delivered in two directions, i.e. alternates. Furthermore, the term "pulsed" as used in the context of direct current is used to describe a periodic current which changes in amplitude. The pulses can occur individually or in a series.

At the frequencies used with the device, low effective impedances occur, and the current may be supplied to the wound without inflicting pain on the treated patient or animal. Without being bound by theory, it is believed that the use of high frequency pulsed DC signals sufficiently mimics the natural electrical stimulations occurring at or in the wound while at the same time ensuring sufficient penetration of the signal through the skin-barrier.

Experiments using an AC voltage source at 2Vpp have shown that a low effective electrical impedance of the skin may be observed when such current pulses (high frequency pulses) are applied to the skin. Such low impedance values have been observed to be in the range of 144 to 173 ohms for pulses of 50 to 500 kHz (2 to 20μs). The same effect is thus expected for a DC pulses of the same frequencies. On the contrary, impedance values of the skin are much higher when the frequency of the current pulses are lower, such as 1000 to 20,000 ohms for 1 Hz to 1 kHz. The device of the present disclosure therefore advantageously allows a greater current flow through the skin, which, without being bound by theory, improves the therapeutic effect. In addition, the device of the present disclosure may allow for the determination of the approximated voltage drop over the wound, without having to take into account differences in the impedance in the skin surrounding the wound, which can vary greatly.

Furthermore, the experiments using the AC voltage source at 2Vpp show that current pulses having a frequency in the range of 50 to 500 kHz are pain free, whereas pulses having a frequency of 0,8 to 3 kHz result in a direct sense of pain. The device of the present disclosure may therefore advantageously also allow for a pain free application of a therapeutic current and may be pain free even when applied for many hours, such as 0.5-12 hours, 0.5-8 hours, or 0.5-4 hours.

The current pulses may have a frequency in the range of 50 to 500 kHz, 60 to 400 kHz, 70 to 300 kHz, or 80 to 200 kHz.

The pulsed direct current has a duty cycle in the range of 1-10%. In a preferred embodiment, the pulsed direct current has a duty cycle in the range of 1-4%.

The pulsed direct current has a duty cycle in the range of 1-10%. In the context of the present invention, a duty cycle is to be understood as the percentage of the ratio of pulse duration or pulse width to a total period of a waveform. A duty cycle in the range of 1-10% may result in a suitable electrotherapy of a wound, especially in combination with the DC pulses disclosed above. Such a duty cycle may also prevent excessive pH build-up and the generation of heat in the treated wound and tissue.

. Further, by using a duty cycle, power may be saved which increases battery life and extends total treatment time. In a preferred embodiment, the pulsed direct current has a duty cycle in the range of 1-4%. In another embodiment, the pulsed direct current has a duty cycle of 2%. The inventors have found that a pulsed direct current of at least 50 kHz, such as 100 kHz, and a duty cycle of 1-10% is suitable for electro-therapy of wounds.

In the context of the present inventive concept, the short period in which no voltage is outputted may be any period deemed suitable for a course of treatment. The no voltage period may for example be decided based on the state, e.g. size or placement, of the wound, the patient's health, the battery lifetime, and a combination of these.

The electro-therapeutic device of the present inventive concept is suitable for wound healing treatment. The course of treatment may be constructed as a series of treatment time periods and no voltage, or downtime periods.

The treatment time period may be 5 minutes - 10 hours, 5 minutes - 5 hours, 5 minutes - 1 hour, 15 minutes - 45 minutes, or 20 minutes - 40 minutes, e.g. 30 minutes. The treatment time period being a period of time where a current for electrotherapy is applied to the wound.

The no voltage period may be 5 minutes - 10 hours, 5 minutes - 5 hours, 5 minutes - 1 hour, 15 minutes - 45 minutes, or 20 minutes - 40 minutes, e.g. 30 minutes. The down time period may have the same duration as the treatment time period. The down time period being a period of time where no current for electro therapy is applied to the wound.

Allowing pauses in electrotherapy allows for the wound to discharge a charge which may have build-up in the wound during the electrotherapy.

In an embodiment, the average current applied to the wound through the stimuli electrodes is in the range of approximately 1-10 mA.

Such a current may not be tissue damaging and may result in a current suitable for electrotherapy. Limiting the device to these output levels mitigates applying harmful currents to wounds.

In an embodiment, the pulsed direct current applied to the wound through the stimuli electrodes is with a frequency in the range of 50 to 500 kHz. In another embodiment, the pulsed direct current applied to the wound through the stimuli electrodes is with a frequency in the range of 100 to 500 kHz.

The inventors have found that the use of frequency in the range of 50 to 500 kHz, such as in the range of 100 to 500 kHz is effective in wound healing while at the same time causing a minimum amount of stress and pain to the patient being treated.

The present disclosure provides a device for electro-therapy. The device comprises a controlled voltage source connected to at least two stimuli electrodes, the stimuli electrodes being configured to be electrically in contact with skin surrounding a wound and the controlled voltage source being configured to output a pulsed direct current with a frequency of at least 50 kHz, and wherein the pulsed direct current has a duty cycle in the range of 1-10%.

In the context of the present disclosure, skin surrounding a wound is understood as any area of skin adjacent to or which abuts the wound. It is understood as skin which is at maximum 50 mm from the edge of a wound.

The electrodes may be of any material with good conductive properties (conductor), such as a metal such as platinum, gold, cobber, silver, iron or led, or an alloy, or other conductive materials such as carbon.

The electrical contact between the electrodes and the skin surrounding a wound may be any kind of contact made between an electrode and the skin surrounding the wound, which enables the conduction of a current. For example, it may be a direct contact between a conductor of the electrode and the tissue. It may also be indirect, such as when a conductive medium is laid between the electrode and the skin surrounding the wound. Without limiting the present disclosure to a specific conductive medium, an example of such a conductive medium may be conductive hydrogels, which are well known from their use in electrocardiography and in ultrasound applications. The conductive medium may also be a solution of an electrolyte. For a given wound, a conductive medium may cover the skin surrounding the wound or cover a portion of the skin surrounding the wound.

In the context of the present disclosure, electrodes that are configured to be electrically in contact with skin surrounding a wound should be understood as electrodes which have a surface or which can be configured to have a surface suitable for being contact with skin surrounding a wound, which surface can make an electrical contact to the skin surrounding a wound. A suitable surface is substantially flat and/or may be curved or bend to a shape that has a surface fitting the curvature of the skin, as is known from patches such as band-aid or cardiac monitoring electrodes. The electrodes may have any shape, if the shape results in a suitable surface. The surface should at least in part be of conductive material, such that electricity can be conducted through the electrodes and the skin the electrodes are arranged on. To improve electrical contact, a gel layer may be arranged between the electrode and the skin.

The device of the present disclosure allows for a current to run through a wound, when the stimuli electrodes of the device are put into electrical contact with the skin surrounding the wound. The device supplies the current through the stimuli electrodes, and the current may run from one of the stimuli electrodes, through the skin, through the wound, through the skin again, and to another stimuli electrode. When a current is applied to the wound by the device, the electrical field formed in the wound may accelerate wound healing, and as such the device may provide electrotherapy for a wound.

In an embodiment, the average current outputted from the controlled voltage source is limited to the range of 1 to 10 mA.

Such current levels are generally not tissue damaging and results in a current suitable for electrotherapy. The device may be limited to these output levels in order to mitigate applying harmful currents to wounds. The peak current levels may be greater than e.g. 10 mA. The average current level may be 2 to 8, 3 to 7, or 4 to 6 mA.

In an embodiment, the average peak voltage outputted from the controlled voltage source is limited to the range of 0.1 to 20 V.

Such voltage levels are generally not tissue damaging and results in a voltage level suitable for electrotherapy. The device may be limited to these output levels in order to mitigate applying harmful currents to wounds. The peak current levels may be greater than e.g. 10 mA. The average peak voltage level may be 0.2 to 18V, 0.3 to 17V, 0.4 to 16V or 0.5 to 15V.

The inventors have found that a pulsed direct current of at least 50kHz that has an average current output of 1 to 10 mA and an average peak voltage output of 0.1 to 20 V, may be especially suitable for electro-therapy of wounds.

In an embodiment, the controlled voltage source is configured to output a pulsed direct current with a frequency in the range of 50 to 500 kHz. In another embodiment, the controlled voltage source is configured to output a pulsed direct current with a frequency in the range of 100 to 500 kHz.

The inventors have found that such a current result in a suitably very low impedance in a model wound while not inflicting any pain on the mammal or patient being treated with the current. The inventors have found that the use of an intermittent electrical signal, i.e. a monophasic pulsed direct current with a duty cycle in the range of 1-10% and at a frequency of at least 50 kHz is sufficient for transcending the skin barrier and provides superior results in wound healing.

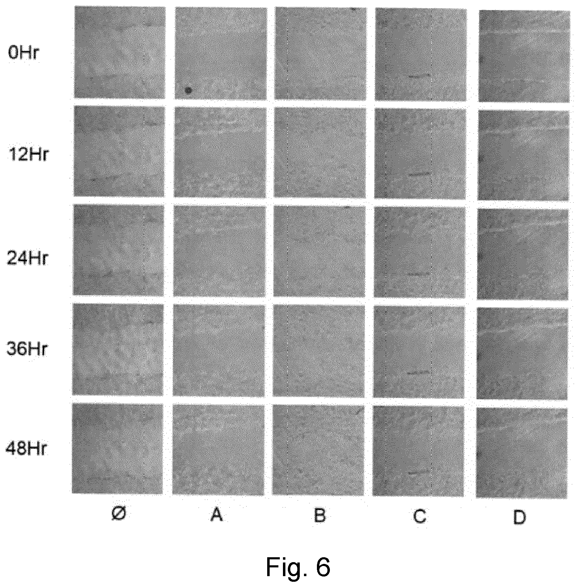

Without being bound by theory, it is believed that the use of mono pulsed DC with a frequency of at least 50 kHz fully avoids the discomfort, such as itching, skin reactions or muscle activation, which is normally associated with biphasic DC or lower frequency currents through skin. Furthermore, the pause in treatment, i.e. the inactive period in which no current is provided, ensures optimal battery life and avoids that the treated cells are overly stimulated. Data to support these observation is shown and described in Example 1 and Example 2 as well as Figures 6, 7 and 8 provided herewith.

**Brief description of the figures**

In the following, embodiments of the present invention will be described with reference to the enclosed non-binding drawings.

Figure 1 is a flowchart illustrating an embodiment of the method of using the device according to the present disclosure;
Figure 2 shows a device, which may be used to carry out the method of using the device of the present disclosure;
Figure 3 shows the patch of figure 2 in an exploded view;
Figure 4 shows the patch of figure 2 and 3 in an assembled condition;
Figure 5 shows a planar cross-section of the device of figure 2, 3, and 4, when applied to skin;
Figure 6 shows representative light microscope images of scratch assays in monolayers of HaCaT cells exposed to electrical stimulation over 48 hours;
Figure 7 shows representative light microscope images of scratch assays in monolayers of HaCaT cells exposed to electrical stimulation over 48 hours;
Figure 8 shows a graph of cell migration ability of HaCaT cells exposed to electrical stimulation over 48 hours.

**Detailed description of the invention**

The present invention will be described below relative to specific embodiments. Those skilled in the art will appreciate that the present invention may be implemented in a number of different applications and embodiments and is not specifically limited in its application to the particular embodiment depicted herein.

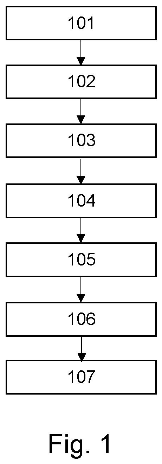

Figure 1 is a flow diagram illustrating relevant steps in how the device according to the present disclosure may be used. In a first step 101, two stimuli electrodes are provided. In a second step 102, the two stimuli electrodes are brought into electrical contact with a discrete part of the skin surrounding the wound. In a third step 103, a pulsed direct current with a frequency of at least 5 kHz, such as at least 50 kHz, is applied to the wound through the two stimuli electrodes. In an optional fourth step 104, at least two sense electrodes are provided. In an optional fifth step 105, the sense electrodes are arranged on discrete parts of the skin surrounding the wound. In an optional sixth step 106, a voltage drop over the wound is measured via the sense electrodes. In an optional seventh step 107, the voltage outputted to the wound via the stimuli electrodes is regulated based on the measured voltage drop measurement.

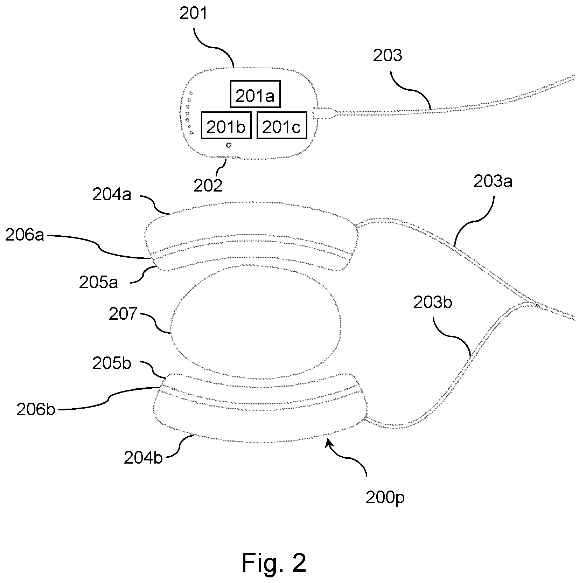

Figure 2 shows a device, which may be used to carry out an electro-therapy. A central unit 201 comprising a control module 201a, a voltage measurement circuit 201b, and a current measurement circuit 201c, is arranged close to a wound 207 and is connected via a cable 203 to two stimuli electrodes 204a and 204b and two sense electrodes 205a and 205b. The cable 203 splits into two cables 203a and 203b, each connected to a sense electrode 205a or 205b and a stimuli electrode 204a or 204b and optionally supporting structures 206a and 206b. A sense electrode 205a, a stimuli electrode 204a and a supporting structure 206a forms a patch 200p.

The central unit 201 may be controlled from at least a button 202 on the central unit 201. The electrode pairs 204a and 205a, and 204b and 205b are respectively connected to two supporting structures 206a and 206b arranged on opposite sides of the wound 207, the sense electrodes 205a and 205b being closest to the wound 207. In this figure, the supporting structures 206a and 206b are illustrated as being transparent, to show the configuration of the electrodes. In operation, electricity is outputted by the central unit 201 and conducted via the cables 203, 203a, and 203b, through the stimuli electrode 204a and 204b, and across the wound 207. The voltage drop across the wound 207 is measured via the sense electrodes 205a, 205b and the central unit 201 by the voltage measurement circuit. The control module 201a may calculate a suitable output voltage based on the measurements of voltage and current. A distance, such as the diameter of the wound, may be entered using the button 202, or by a wireless interface. The entered value can be used by the central unit 201 to calculate suitable voltage output levels.

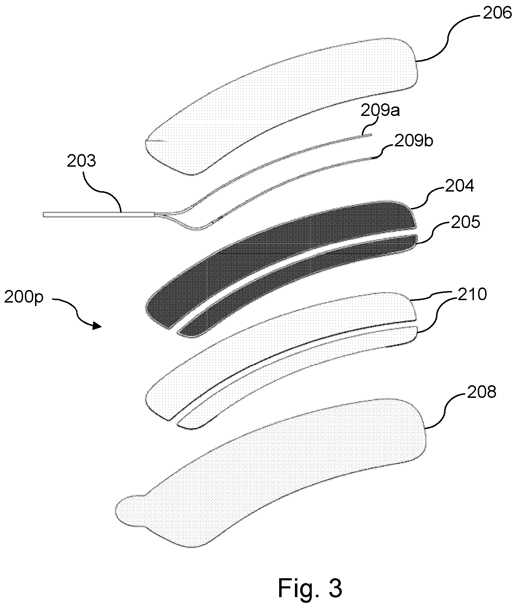

Figure 3 shows the patch 200p of figure 2 in an exploded view. The patch 200p comprises the supporting structure 206 and electrodes 204 and 205. A cable 203 comprises two wires 209a and 209b, that are unshielded. When the patch 200p is assembled, the two wires 209a and 209b are respectively in electrical contact with a stimuli electrode 204 and a sense electrode 205 and are sandwiched between the supporting structure 206 and the electrodes 204 and 205. A conductive gel layer 210 is optionally located between each respective electrode 204 and 205 and an optional removable film 208.

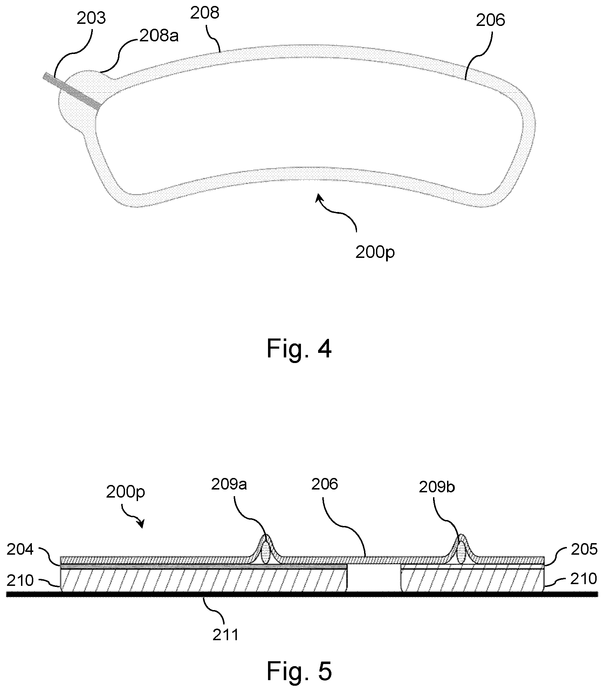

Figure 4 shows the patch 200p of figure 2 and 3 in an assembled condition. The removable film 208 optionally has a notch 208a to enable easy removal of the film. This embodiment may be cut, for instance, across the width of the patch, such that the length of the patch is reduced. Ideally, the length of the patch is cut, so that it fits roughly with the dimensions of skin surrounding the wound to be treated.

Figure 5 shows a planar cross-section of the patch 200p of figure 2, 3, and 4 in an assembled form, where the removable film 208 has been removed, and the conductive gel layer 210 is contacting the surface of the skin 211 surrounding the wound (not shown).

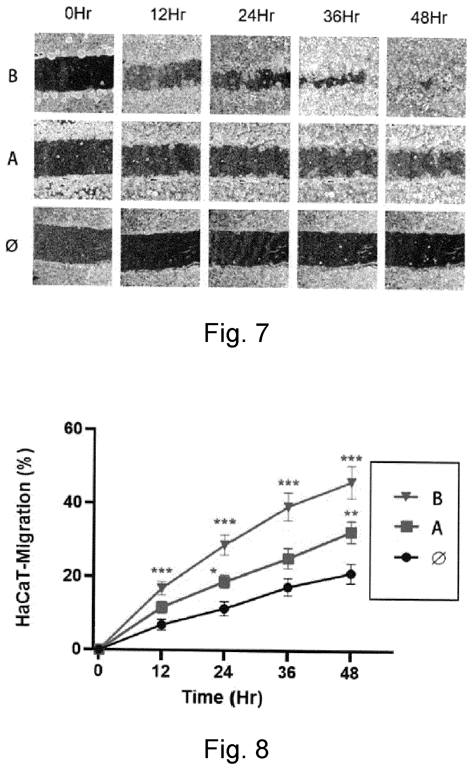

Figure 6 shows representative light microscope images of the scratch assay described in Example 2 in which monolayer HaCaT cells were exposed to electrical stimulation under different conditions over 48 hours. The different conditions tested were A, pulse frequency of 0 kHz, duty cycle of 100% and electric field of 200 mV/mm; B, pulse frequency of 100 kHz, duty cycle of 2% and electric field of 200 mV/mm; C, pulse frequency of 100 kHz, duty cycle of 4% and electric field of 200 mV/mm; and D, pulse frequency of 100 kHz, duty cycle of 10% and electric field of 150 mV/mm. The control Ø represents untreated, i.e. unstimulated, cells. Each condition was tested in triplicates and the images shown in Figure 6 represents a representative image of the general cell behaviour observed for each condition. Images were acquired after 0 hours, 12 hours, 24 hours, 36 hours and 48 hours of electrostimulation. For all conditions, the electrical stimulation was orthogonal (top to bottom) to the scratches, the scratches being present in the middle of the image. For the control group Ø, the cell-free area, i.e. the scratch, in the middle of the image has the same size over the entire 48 hours. For conditions C and D, the cell-free area in the middle of the images shows only slight reduction in size, i.e. wound healing under these conditions were slow and less efficient. For condition A, a visible change in the size of the cell-free area was observed over the course of treatment. Cells treated with condition B by far showed the largest reduction in the size of the cell-free area, thus representing the best condition for wound healing. The healing properties of conditions A and B are shown more clearly in Figure 7.

Figure 7 shows representative light microscope images of the scratch assay described in Example 2 with monolayer HaCaT cells exposed to electrical stimulation under stimulated conditions (A and B) and unstimulated condition (Ø) over 48 hours. The scratch area or cell-free area represents the wound and is seen in the middle of the image as a darker area. During electrical stimulation, the size of the cell-free area is reduced in the treated cells (A and B), whereas for the unstimulated cells (Ø) this area remains unchanged in size. Furthermore, the cells treated under condition B (pulse frequency of 100 kHz, duty cycle of 2% and electric field of 200 mV/mm) display the fastest and most efficient wound healing as shown from the substantial reduction in the cell-free area which is visible already after 24 hours. After 48 hours of treatment under condition B, the cell-free area is almost completely reduced. Condition A displays a slower and less pronounced wound healing.

Figure 8 shows a graph of the cell migration ability of HaCaT cells treated with conditions A (solid line with squares) and B (solid line with triangles) and for untreated cells Ø (solid line with circles). The graph depicts the change in cell-free area over time, which is calculated using the Image J software, an open-source image processing program used to assess wound closure by tracing the wound perimeter and calculating percentage closure. The width of the scratches at 0 hours is 100%. As seen in Figure 8, the change in cell-free area over time, i.e. rate of wound healing, is highest for HaCaT cells treated under condition B, which is evident from the approximately 50 % change in cell-free area at 48 hours.

## Examples

Example 1: Demonstration of low impedance and no pain at high AC frequencies in a simulated wound.

Two electrodes were arranged on dry skin on a person's arm, the distance between the electrodes being 50 mm. The electrodes were connected in series to a signal generator with a 50 ohm output impedance. A 100 ohms resistor was inserted in series into the circuit as a measurement resistor for current measurements. A simulated wound was made on the arm by applying a thick layer of Cefar Electrogel to the skin between the electrodes. The Cefar Electrogel was applied on the skin to form a roughly circular gel layer, the gel layer being about 5 mm from each of the electrode's edges. Electrical measurements were made by connecting an oscillometer in series, the first and the second lead of the oscillometer being respectively connected right before and after the 100 ohms resistor. Briefly, the measurements showed that the AC resistance of the simulated wound is strongly frequency dependent and ranges from about 20 kOhm at 1Hz to <150 Ohm in the range 100 kHz to 1 MHz, and that it has a DC resistance which is voltage dependent and is in the range of 40 kOhm (at 20V) to 263 kOhm (at 1V). After several hours of AC measurements, there is no irritation to the skin, while the DC (non-pulsed) measurements, which took only 10 minutes, and which measured currents below 0.5mA, nevertheless resulted in red and irritated skin under the positive electrode, as well as some discomfort in the skin already at DC voltage/currents above 15V/150$\mu$A. At AC frequencies of 0.8 to 3 kHz, direct sensation of pain could be felt. Below in table 1 are given impedance values of the simulated wound at different AC frequencies. The table also shows the AC frequencies at which a direct sense of pain is felt.

Table 1.

| AC Frequency [kHz] | Impedance in model wound (ohms) | Pain | | AC Frequency [kHz] | Impedance in model wound (ohms) | Pain |
|---|---|---|---|---|---|---|
| 0,001 | 19850 | NO | | 10 | 247 | NO |
| 0,01 | 16517 | NO | | 20 | 205 | NO |
| 0,02 | 15235 | NO | | 30 | 192 | NO |
| 0,03 | 12350 | NO | | 40 | 181 | NO |
| 0,04 | 9850 | NO | | 50 | 173 | NO |
| 0,05 | 8941 | NO | | 60 | 166 | NO |
| 0,06 | 8183 | NO | | 70 | 163 | NO |
| 0,07 | 7850 | NO | | 80 | 163 | NO |
| 0,08 | 7257 | NO | | 90 | 159 | NO |
| 0,09 | 6517 | NO | | 100 | 155 | NO |
| 0,1 | 6302 | NO | | 200 | 148 | NO |
| 0,2 | 3696 | NO | | 300 | 148 | NO |
| 0,3 | 2667 | NO | | 400 | 144 | NO |
| 0,4 | 1978 | NO | | 500 | 144 | NO |
| 0,5 | 1719 | NO | | 600 | 144 | NO |
| 0,6 | 1463 | NO | | 700 | 144 | NO |
| 0,7 | 1201 | NO | | 800 | 144 | NO |
| 0,8 | 1116 | **YES** | | 900 | 144 | NO |
| 0,9 | 1013 | **YES** | | 1000 | 144 | NO |
| 1 | 986 | **YES** | | 2000 | 153 | NO |
| 2 | 554 | **YES** | | 3000 | 190 | NO |
| 3 | 425 | **YES** | | 4000 | 263 | NO |
| 4 | 360 | NO | | 5000 | 192 | NO |
| 5 | 326 | NO | | 6000 | 218 | NO |
| 6 | 300 | NO | | 7000 | 250 | NO |
| 7 | 281 | NO | | 8000 | 200 | NO |
| 8 | 263 | NO | | 9000 | 188 | NO |
| 9 | 260 | NO | | 10000 | 188 | NO |

Conclusion: levels of current and voltage suitable for electrotherapy may be provided painlessly to a simulated wound through the skin using an AC signal having a frequency of at least 10 kHz. Pulsed DC signals similar to the AC signals are expected to generally behave in similar way, and may therefore also be used in electrotherapy.

Example 2: *In vitro* studies on HaCaT cells stimulated electrically versus control

The electrical stimulation system setup

For the basic principle of the electrical stimulation system, the protocol published by Song *et al.* in 2007 (doi:10.1038/nprot.2007.205) was used as a starting point. The key parts of the stimulation system included cell culture dishes with special structures, including cell culture chambers, salt bridges to transport the electrons, electrical stimulation devices to generate the electrical current, and scaffolding to hold everything in place. The purpose of this setup was to generate the target electric field in the cell culture environment. The protocol of Song *et al.* 2007 was adapted to accommodate the special requirements of the study, which included long duration of electrical stimulation, microscopic examination, etc.

Cell culturing and method of scratch assay

The HaCaT cells were cultivated in monolayers in thin chambers to avoid cell damage caused by excessive heating of the medium by the electric field. The cells were seeded in the middle of the custom-made petri dishes. Normally, the culture concentration of HaCaT cells is $1 \times 10^4/cm^2$. To make the cells confluent as quickly as possible in a limited space, the concentration of the cell suspension used for seeding was $95 \times 10^4/cm^2$. Viability of the cells under the provided experimental conditions were confirmed by trypan blue staining to be greater than 95%.

Normally, the cell-free area is generated by using a pipette tip to scratch a wound through the center of the Petri-dish, however, for the present experimental setup the use of tips led to issues with the edges of the scratches being irregular, the cells being easier to lift in layers around the edges and the cells at the edges of the scratches breaking. To circumvent these issues, a self-adhesive silicone tape was used instead of tips. The silicone tape was cut into strips of equal width (0.5-0.7mm) and adhered to the cell culture area in advance. Once sufficient cell growth and cell adhesion was achieved, the tape was removed and satisfactory cell-free areas were generated.

The effect of electrical stimulation on cell migration

To explore the effects of different modes of electrical stimulation on HaCaT cell migration, electrical signals with different parameters (Table 2) were applied to the monolayer of HaCaT cell culture. Stimulation (Conditions A, B, C, D) and no stimulation (control group Ø) was applied to a monolayer of HaCaT cell culture using Ag/AgCl electrodes connected via agar salt bridges, to prevent electrode products from entering cultures.

Table 2 Parameters tested on HaCaT cell culture systems

| Condition | Pulse frequency (kHz) | Duty cycle (%) | Electric field (mV/mm) |
|-----------|----------------------|----------------|------------------------|
| A | 0 | 100 | 200 |
| B | 100 | 2 | 200 |
| C | 100 | 4 | 200 |
| D | 100 | 10 | 150 |

The scratches or cell-free area in the culture system were photographed at 0, 12, 24, 36 and 48 hours after the start of electrical stimulation. At each time point, three photos of each cell-free area were taken (Figure 6) and the cell-free area was calculated by Image J software. The original microscope magnification was 40x.

The following formula was used to calculate the migration rate of each group of cells at different time points to evaluate the migration ability:

$$\text{Migration Rate} = \frac{\text{Cell-free area(0H)} - \text{Cell-free area(nH)}}{\text{Cell-free area(0H)}} \times 100 \quad, n = 12, 24, 36, 48$$

Compared with the control group (Ø), the migration rate of the experimental groups (A, B) showed an increasing trend (Figure 7 and 8). HaCaT cells cultures exposed to the electrical stimulation generated by Condition B (100 kHz, 2% duty cycle, 200 mV/mm) showed the most significant increase in migration ability, i.e. the fastest and most efficient wound healing. As seen from Figure 7, the wound closed substantially after only 24 hours of electrical stimulation treatment and was almost completely closed by 48 hours of electrical stimulation treatment. As seen from Figure 8, both conditions B and A markedly enhanced the rate of wound closure as compared to the control (Ø). Furthermore, condition B significantly outperformed condition A (normal DC stimulation) in that condition B closed wounds roughly 50 % faster (*p< 0.05, **p<0.01, ***p<0.001).

Statistics

Results are presented as meant standard error of mean (SEM). Each test conditions were performed three independent times (N = 3) measured as triplicate or more (technical replicates, n≥3). Statistical analyses were performed using the GraphPad Prism Software (Version 8.0.2, El Camino Real, USA). Data sets were compared by the Kruskal-Wallis H test. $\alpha = 0.05$ was set as the maximum type I error rate. * < 0.05, ** < 0.01 , *** < 0.001 were used to classify P values in comparisons with the control group.

Conclusions

The cell system described in this example meets the requirements of testing cell migration ability.

Electrical stimulation provided under conditions A and B showed a trend of improving the cell migration ability, with the changes in condition B, i.e. a pulse frequency of 100 kHz, a duty cycle of 2% and an electric field of 200 mV/mm, being the most significant.

Based on the *in vitro* studies presented above, it is evident that the electrical stimulation as provided in accordance with the present inventive concept provides superior wound healing properties. Furthermore, in accordance with the data presented in Example 1, it is believed that the electrical stimulation conditions tested herewith cause minimum adverse effects to the patient during the electrotherapy. Thus, it is believed that an optimum stimulation algorithm consisting of high frequency pulses of at least 50 kHz or 100 kHz with a low duty cycle (<10%), results in the highest possible electrical field (EF) strength across a wound at a minimum battery power consumption and with minimum power dissipation in the skin. This maximizes battery life, establishes the desired EF of 50 - 200mV/mm across the wound and fully avoids discomfort, such as itching, skin reactions or muscle activation, associated with DC or lower frequency currents through skin.

**Claims**

1. A device for electro-therapy, the device comprising a controlled voltage source connected to at least two stimuli electrodes (204a, 204b), the stimuli electrodes (204a, 204b) being configured to be electrically in contact with skin having a wound, **characterised in that** the stimuli electrodes (204a, 204b) are configured to be electrically in contact with skin from the edge of the wound to a distance at maximum 50 mm from the edge of the wound, and **in that** the controlled voltage source is configured to output a pulsed direct current through the wound with a frequency of at least 50 kHz, and **in that** the pulsed direct current has a duty cycle in the range of 1-10%.

2. The device according to claim 1, wherein the average current outputted from the controlled voltage source is limited to the range of 1 to 10 mA.

3. The device according to claim 1 or 2, wherein the average peak voltage outputted from the controlled voltage source is limited to the range of 0.1 to 20 V.

4. The device according to any one of the preceding claims, wherein the pulsed direct current has a duty cycle in the range of 1-4%.

5. The device according to any one of the preceding claims, wherein the current is a monophasic pulsed direct current.

6. The device according to any one of the preceding claims, wherein the controlled voltage source is configured to output a pulsed direct current with a frequency in the range of 50 to 500 kHz.

7. The device according to any one of the preceding claims, wherein the controlled voltage source is configured to output a pulsed direct current with a frequency in the range of 100 to 500 kHz.

**Patentansprüche**

1. Vorrichtung zur Elektrotherapie, wobei die Vorrichtung eine gesteuerte Spannungsquelle umfasst, die mit mindestens zwei Stimulationselektroden (204a, 204b) verbunden ist, wobei die Stimulationselektroden (204a, 204b) dazu ausgelegt sind, elektrisch mit Haut in Kontakt zu sein, die eine Wunde aufweist, **dadurch gekennzeichnet, dass** die Stimulationselektroden (204a, 204b) dazu ausgelegt sind, elektrisch mit Haut vom Rand der Wunde in einem Abstand von maximal 50 mm vom Rand der Wunde in Kontakt zu sein, und dadurch, dass die gesteuerte Spannungsquelle dazu ausgelegt ist, einen gepulsten Gleichstrom mit einer Frequenz von mindestens 50 kHz durch die Wunde auszugeben, und dadurch, dass der gepulste Gleichstrom einen Duty Cycle im Bereich von 1-10 % hat.

2. Vorrichtung nach Anspruch 1, wobei der durchschnittliche Strom, der von der gesteuerten Spannungsquelle ausgegeben wird, auf den Bereich von 1 bis 10 mA begrenzt ist.

**3.** Vorrichtung nach Anspruch 1 oder 2, wobei die durchschnittliche Spitzenspannung, die von der gesteuerten Spannungsquelle ausgegeben wird, auf den Bereich von 0,1 bis 20 V begrenzt ist.

**4.** Vorrichtung nach einem der vorhergehenden Ansprüche, wobei der gepulste Gleichstrom einen Duty Cycle im Bereich von 1-4% hat.

**5.** Vorrichtung nach einem der vorhergehenden Ansprüche, wobei der Strom ein einphasiger gepulster Gleichstrom ist.

**6.** Vorrichtung nach einem der vorhergehenden Ansprüche, wobei die gesteuerte Spannungsquelle dazu ausgelegt ist, einen gepulsten Gleichstrom mit einer Frequenz im Bereich von 50 bis 500 kHz auszugeben.

**7.** Vorrichtung nach einem der vorhergehenden Ansprüche, wobei die gesteuerte Spannungsquelle dazu ausgelegt ist, einen gepulsten Gleichstrom mit einer Frequenz im Bereich von 100 bis 500 kHz auszugeben.

**Revendications**

**1.** Dispositif d'électrothérapie, le dispositif comprenant une source de tension commandée connectée à au moins deux électrodes de stimulation (204a, 204b), les électrodes de stimulation (204a, 204b) étant configurées pour être électriquement en contact avec la peau présentant une plaie, **caractérisé en ce que** les électrodes de stimulation (204a, 204b) sont configurées pour être électriquement en contact avec la peau depuis le bord de la plaie jusqu'à une distance maximale de 50 mm du bord de la plaie, et **en ce que** la source de tension commandée est configurée pour émettre un courant continu pulsé à travers la plaie à une fréquence d'au moins 50 kHz, et **en ce que** le courant continu pulsé a un rapport cyclique dans la plage de 1 à 10 %.

**2.** Dispositif selon la revendication 1, le courant moyen émis par la source de tension commandée étant limité à la plage de 1 à 10 mA.

**3.** Dispositif selon la revendication 1 ou 2, la tension moyenne de crête émise par la source de tension commandée étant limitée à la plage de 0,1 à 20 V.

**4.** Dispositif selon l'une quelconque des revendications précédentes, le courant continu pulsé ayant un rapport cyclique dans la plage de 1 à 4 %.

**5.** Dispositif selon l'une quelconque des revendications précédentes, le courant étant un courant continu pulsé monophasique.

**6.** Dispositif selon l'une quelconque des revendications précédentes, la source de tension commandée étant configurée pour délivrer un courant continu pulsé avec une fréquence dans la plage de 50 à 500 kHz.

**7.** Dispositif selon l'une quelconque des revendications précédentes, la source de tension commandée étant configurée pour délivrer un courant continu pulsé avec une fréquence dans la plage de 100 à 500 kHz.

Fig. 1

Fig. 2

Fig. 3

**Fig. 4**

**Fig. 5**

Fig. 6

Fig. 7

Fig. 8

**EP 4 135 832 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005032652 A1
- WO 2009060211 A1
- WO 02098502 A
- WO 2008013936 A
- US 2004267333 A1
- US 6535767 B1
- US 2009132010 A1
- US 5018524 A